# EUROPEAN PATENT SPECIFICATION

(11) **EP 3 013 252 B1**
(45) Date of publication and mention of the grant of the patent: **07.03.2018**
(21) Application number: 14739327.6
(22) Date of filing: 24.06.2014
(51) Int. Cl.: A61B 17/04, A61B 17/08, A61B 17/12, A61B 17/122, A61B 17/128, A61B 17/10

(54) **HEMOSTASIS DEVICE WITH ONE WAY TRAP**
HÄMOSTASEVORRICHTUNG MIT EINWEGFALLE
DISPOSITIF D'HÉMOSTASE AVEC PIÈGE À UNE VOIE

(30) Priority: 25.06.2013 US 201361839009 P
(43) Date of publication of application: 04.05.2016
(73) Proprietor: Boston Scientific Scimed, Inc., Maple Grove, MN 55311 (US)
(72) Inventor: RAYBIN, Samuel, Marlborough, MA 01752 (US); RYAN, Shawn, Upton, MA 01568 (US); SMITH, Paul, Smithfield, RI 02917 (US)
(74) Representative: Vossius & Partner Patentanwälte Rechtsanwälte mbB
(86) International application number: PCT/US2014/043890
(87) International publication number: WO 2014/210019

(56) References cited:
- WO-A2-2007/017872
- US-A1- 2002 062 130
- US-A1- 2005 075 665
- US-A1- 2007 049 967
- US-A1- 2009 157 101
- US-A1- 2012 253 362

## Description

### Background

Pathologies of the gastrointestinal ("GI") system, the biliary tree, the vascular system and other body lumens and hollow organs are often treated through endoscopic procedures, many of which require active and/or prophylactic hemostasis to control bleeding. Hemostasis clips are often deployed via endoscopes to stop internal bleeding by holding together the edges of wounds or incisions to allow natural healing processes to close the wound. Specialized endoscopic clipping devices are used to deploy the clips at desired locations after which the clip delivery device is withdrawn, leaving the clip within the body. Current hemostasis devices, however, are expensive, generally single use, and may be difficult to deploy.

US 2002/062130 A1 discloses a system for delivering a surgical clip to a surgical site within a patient's body to compress body tissue. The system includes an endoscopic device that has an endoscope cap disposed on a distal end of the endoscopic device. A surgical clip is removably disposed on an outside surface of the endoscope cap. A deployment device is associated with the surgical clip for deploying the surgical clip from the endoscope cap to the body tissue that is to be compressed. The surgical clip can be a deformable clip that is deployed in a non deformed configuration, and is then deformed so as to apply compression on selected tissue.

US 2005/075665 A1 discloses an apparatus that gathers a patient's body tissue and then secures the gathered tissue in a reduced area utilizing a securing structure. The securing structure mainly resides on one side of the tissue to minimize or eliminate both foreign material and the amount of manipulation or activity on the other side of the tissue.

US 2007/049967 A1 discloses a vascular closure device which comprises a retrievable sheath-delivered contractible clip device with structural radial or terminal members with terminal and non-terminal hooks that engage the vessel wall. The device is delivered on the outside of a sheath. Closure of the tissue opening can be effected by the feet of the clip engaging the puncture, aperture, or wound edges and memory characteristics of the device cause a contraction of the members. The device can be delivered and recovered by an intravascular sheath.

US 2009/157101 A1 discloses a tissue closure system. A closure element includes a coiled body and a plurality of tissue engaging portions. The tissue engaging portions are disposed about at least a portion of the coiled body. The coiled body is formed of a resilient material.

WO 2007/017872 A2 discloses a system for surgical fastening including a surgical fastening device configured to deploy one or more surgical fasteners. The surgical fastener has a crown from which extend two or more prongs. The prongs are parallel to the axis of the crown and each prong is provided with one or more barbs.

US 2012/253362 A1 discloses a compression ring to grip and compress a body structure. A resilient ring-shaped body defines a compression channel, and an elongated axially rigid gripping member extends diametrically across the through-opening. The gripping member can rest on a flange on the opposite side of the through-opening or engage with a second gripping member that extends diametrically across the through-opening from the opposite side of the ring.

A tissue clipping device as defined by claim 1 is provided. The dependent claims define embodiments.

In one aspect, the present disclosure is directed to a tissue clipping device, comprising a first body extending from a proximal end to a distal end and including a first channel extending therethrough and a plurality of first arm elements, each of the plurality of first arm elements extending radially into the first channel from a first end coupled to the first body to a second end proximal of its first end so that tissue drawn into a distal end of the first channel proximally beyond the second end of the plurality of first arm elements is compressed between each of the plurality of first arm elements and retained in the first channel.

In another aspect, the present disclosure is directed to a system for clipping tissue, comprising an insertion member extending longitudinally from a proximal end to a distal end and including a lumen extending therethrough and a first clip slidably received within the lumen and extending from a first end to a second end and including a first channel extending therethrough, the first clip including a plurality of first arm elements, each of the plurality of first arm elements extending radially into the first channel between a first part coupled to a radially outer wall of the first clip and a proximal end proximal to the first part so that tissue drawn into a distal end of the first channel proximally beyond the proximal ends of the first arm elements is compressed between the first arm elements and retained in the first channel.

Also disclosed is a method for clipping tissue, the method not forming part of the claimed invention, comprising inserting into a body lumen a flexible insertion member until a distal end thereof is proximate a first portion of target tissue, drawing the first portion of target tissue into a channel of a first clip received within the distal end of the flexible insertion member until a part of the first portion of target tissue has moved proximally beyond proximal ends of first arm elements extending radially and proximally into the first clip from a distal end of the first clip to capture the first portion of target tissue within the first clip, and releasing the first clip from the flexible insertion member by moving the first clip distally out of the flexible insertion member until a proximal locking feature of the first clip is no longer supported by an interior surface of the flexible insertion member and is released therefrom.

### Brief Description of the Drawings

Fig. 1 shows a longitudinal cross-sectional side view of a device according to an exemplary embodiment of the present disclosure;
Fig. 2 shows a perspective view of a clip according to the device of Fig. 1;
Fig. 3 shows another perspective view of the clip of Fig. 2;
Fig. 4 shows a cross-sectional side view of the clip of Fig. 2;
Fig. 5 shows a cross-sectional side view of the clip of Fig. 2 grasping tissue between arm elements thereof;
Fig. 6 shows a longitudinal cross-sectional view of a device according to another embodiment of the present disclosure;
Fig. 7 shows a cross-sectional side view of the device of Fig. 6 used for tissue approximation;
Fig. 8 shows another cross-sectional side view of the device of Fig. 6 used for tissue approximation;
Fig. 9 shows a cross-sectional view of a clip according to another embodiment of the present disclosure; and
Fig. 10 shows a longitudinal cross-sectional view of a device according to yet another embodiment of the present disclosure not falling within the scope of the claimed invention.

### Detailed Description

The present disclosure may be further understood with reference to the following description and the appended drawings, wherein like elements are referred to with the same reference numerals. The present disclosure generally relates to an endoscopic device and, in particular, to a device comprising a one way trap for capturing tissue. Exemplary embodiments of the present disclosure include a hemostasis clipping device including arm elements which allow tissue to be easily drawn therebetween in a proximal direction, while preventing tissue from moving distally out of the device, so that tissue drawn between the arm elements is captured thereby. Although exemplary embodiments specifically describe a hemostasis clipping device, it will be understood by those of skill in the art that the device of the present disclosure may also be utilized for tissue anchoring, approximation, marking, or any other medical function requiring tissue to be grasped or captured. It should also be noted that the terms "proximal" and "distal," as used herein, are intended to refer to a direction toward (proximal) and away from (distal) a user of the device.

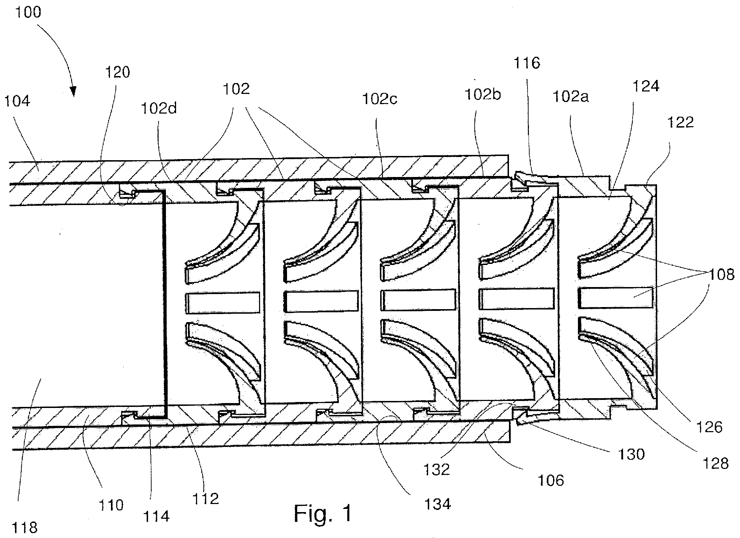

As shown in Fig. 1, a device 100 according to an exemplary embodiment of the present disclosure comprises a plurality of clips 102 received within a distal end 106 of an elongate insertion member 104. Each clip 102 includes a plurality of arm elements 108 cantilevered from a distal end 122 of the clip 102 extending radially into the clip 102 and proximally such that a target tissue may be drawn proximally between the arm elements 108. Each of the arm elements 108 extends from a first end 126 connected to the distal end 122 to a free second end 128 pointed toward a proximal end 116 of the clip 102. Thus, the progress of tissue drawn proximally through the clip 102 is minimally impeded as it slides against the ramped surface formed by the arm elements 108. However, tissue that moves proximally past the free second ends 128 of the arm elements 108 will be held in place within the clip 102 as distally directed force applied by the tissue to the second ends 128 tends to deflect the arm elements 108 distally and further radially toward the center of the clip 102 constricting the opening of the clip 102 and securely grasping the tissue preventing this tissue from moving distally out of the clip 102. Once the target tissue has been captured, a distal most clip 102a is deployed from the tubular member 104 via a deployment member 110 as will be described in more detail below.

The insertion member 104 according to this embodiment is substantially tubular, extending longitudinally from a proximal end (not shown) to the distal end 106 and including a lumen 112 extending therethrough. However, any other appropriate shape may be employed although smoother shapes are preferred as they minimize trauma to surrounding tissue. The insertion member 104 according to this embodiment is sized and shaped for insertion through a working channel of an endoscope and/or a body lumen or hollow organ of a patient. However, those skilled in the art will understand that, for certain applications, an insertion member 104 and the clips 102 may be sized to surround an endoscope leaving the working channel of the endoscope free for other uses. The lumen 112 is sized and shaped to slidably receive the clips 102. The deployment member 110 may also be substantially tubular extending longitudinally from a proximal end (not shown) to a distal end 114 coupleable to a proximal end 116 of a proximal most clip 102d via a coupling element 120. The deployment member 110 also includes a lumen 118 extending therethrough and is sized and shaped to be slidably received within the lumen 112 of the tubular member 104. The deployment member 110 is slidable within the tubular member 104 so that it may be moved distally therethrough to push a distal-most clip 102a from the tubular member 104 when target tissue has been captured thereby. The lumen 118 permits a vacuum or suction force to be applied through the deployment member 110 and through the clips 102 to draw target tissue into the distal most clip 102a between the arm elements 108.

Although embodiments of the present disclosure describe a vacuum or suction force for drawing tissue into the clip, a variety of mechanisms for drawing tissue into the clips 102 may be utilized. For example, tissue may be drawn into the clip 102 by a grasper passed through the clip 102, by a corkscrew-type device screwed into the target tissue or by any other suitable device as would be understood by those skilled in the art. In another embodiment, tissue may be drawn into the clip 102 by the clip 102 distally over a target portion of tissue.

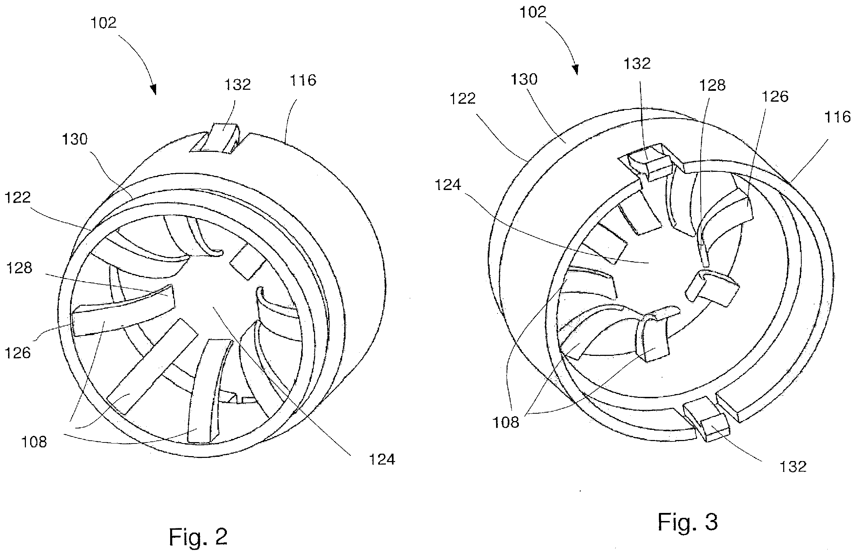

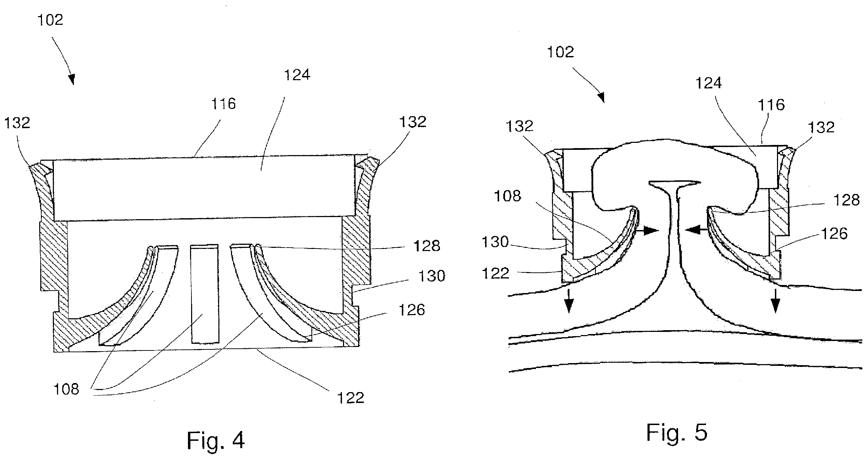

As shown in Figs. 2 - 5, the clip 102 extends from the proximal end 116 to the distal end 122 and includes a channel 124 extending therethrough for receiving the target tissue. The arm elements 108 extend from a first end 126 attached to the distal end 122 to a second end 128 which extends into the channel 124 and is pointed toward the proximal end 116. The arm elements 108 may be curved and/or angled along a length thereof such that tissue may be easily received between the arm elements 108 in a proximal direction. The arm elements 108 may include structures to retain tissue such as, for example, barbs, flaps, spikes or ridges, and may include a tapered or sharpened tip at the second end 128. As shown in Fig. 5, the arm elements 108 may also be deflectable such that when the target tissue is received therebetween, the arms 108 are deflected, applying a compressive force to the tissue received therebetween. In addition, as the tissue itself is naturally pulled distally, the distal end 122 of the clip 102 will also radially compress tissue drawn into the clip 102. The clip 102 may include any number of a plurality of arm elements 108. In one embodiment, the arm elements 108 are equally spaced from one another. The arm elements 108 may be spaced along a perimeter of the clip 102 in any number of configuration. The clip 102 may also include a single arm element 108.

In a further embodiment, the clip 102 may also include a valve which seals the proximal end 116 of the clip 102 so that a vacuum is maintained within the clip 102 after it is deployed. The clip 102 may, for example, include a one-way valve which closes when a certain negative pressure is obtained, a valve which closes when the clip 102 is deployed, or a manually activated valve. The clip 102 may include any of a variety of sealing mechanisms so long as the seal maintains a desired negative pressure within the clip 102 after the clip 102 has been deployed.

The distal end 122 and the proximal end 116 include corresponding distal and proximal locking features 130, 132, respectively, for coupling adjacent clips 102 to one another in a chain-like fashion within the insertion member 104. For example, in this embodiment a distal-most clip 102a is coupled to an immediately proximal clip 102b, the immediately proximal clip 102b is coupled to a next proximal clip 102c and so forth until the clip chain terminates at a proximal-most clip 102d. The distal locking feature 130 in this embodiment includes a groove extending about a perimeter of the distal end 122 which mates with a proximal locking feature 132 formed as a latch extending laterally inward from the proximal end 116 and sized and shaped to engage the groove 130. In another embodiment, the distal locking feature 130 may, for example, includes a latch while the proximal locking feature 132 includes a groove.

The distal and proximal locking features 130, 132 may include any of a variety of interlocking elements so long as the distal and proximal locking features 130, 132 permit adjacent clips to be releasably coupled to one another within the lumen 112 of the insertion member 104. The proximal locking feature 132 in this embodiment is biased radially outward such that when the proximal end 116 of the clip 102 is not restrained by an interior surface 134 of the lumen 112, the proximal locking feature 132 moves radially outward to disengage from the distal locking feature 130 of a proximally adjacent clip 102. The coupling element 120 at the distal end 114 of the deployment member 110 may be substantially similar to the distal locking feature 130 such that the coupling element 120 is engageable with the proximal end 116 of the proximal-most clip 102d.

In some alternative variants that do not form part of the claimed invention and that describe both distal and proximal locking features, where the device 100 includes a single clip 102, the clip 102 may not require a distal locking feature and be frictionally retained in the lumen 112 or may include only a proximal locking feature for releasably coupling the deployment member 110 or other actuating mechanism.

According to a method not forming part of the claimed invention, the device 100 is inserted to a target location in the body, for example, by insertion through a working channel of an endoscope inserted into a body lumen via a natural body orifice. When the distal end 106 of the insertion member 104 is proximate to target tissue to be captured by the clip 102. Thus, as would be understood by those skilled in the art, the insertion member must be sufficiently flexible to be passed through the endoscope even when the endoscope extends along a tortuous path through the body lumen. Tissue is then drawn into the clip 102 by, for example, applying vacuum pressure to the lumen 118. This draws the target tissue into the channel 124 of the clip 102 deflecting the arm elements 108 radially outward until the target tissue extends proximally beyond the free second ends 128 of the arm elements 108. The arm elements 108 are urged radially inward by their natural bias and by the resilience of the target tissue as it pushes distally on the second ends 128 thereof. This distal force applied to the arm elements 108 further compresses the tissue as the arms are rotated distally straightening the arm elements 108 and increasing their extent toward the center of the lumen 118. Once the target tissue has been captured, the clip 102 is released from the insertion member 104 and left in place in the body (e.g., until naturally sloughed off) by advancing the deployment member 110 distally until the proximal locking feature 132 of the distal most clip 102a springs
outward (as it exits the lumen 112) out of engagement with the distal locking feature 130 of the adjacent clip 102b.

Although the method refers to a single clip 102 for capturing tissue, when making use of the device according to the invention, the device 100 includes a plurality of clips 102, the target tissue is captured by the distal-most clip 102a. Once the target tissue has been captured and the distal-most clip 102a has been deployed in the body, the insertion member 104 may be moved to a position adjacent to a second portion of target tissue and the same process may be repeated until all of the clips 102 have been deployed. As described above, the deployment member 110 is coupled to the proximal-most clip 102b such that moving the deployment member 110 relative to the insertion member 104 correspondingly moves the plurality of clips 102 relative to the insertion member 104. Upon moving the distal-most clip 102a distally beyond the distal end 106, the proximal locking feature 132 at the proximal end 116 of the clip 102a is no longer supported by the interior surface 134 of the insertion member 104 and reverts to its biased configuration in which the proximal locking feature 132 extends radially outward disengaging from the distal locking feature 130 of an immediately proximal adjacent clip 102. Once the distal-most clip 102a has been deployed, the immediately proximal adjacent clip 102 becomes the distal-most clip such that the clipping and deploying steps described above may be repeated. The steps described above may be repeated until as many clips 102 as desired have been deployed or until all of the clips 102 have been utilized. In some embodiments, a device including a single clip 102 may deploy the clip 102 in the same manner as described above in regard to the distal-most clip 102a.

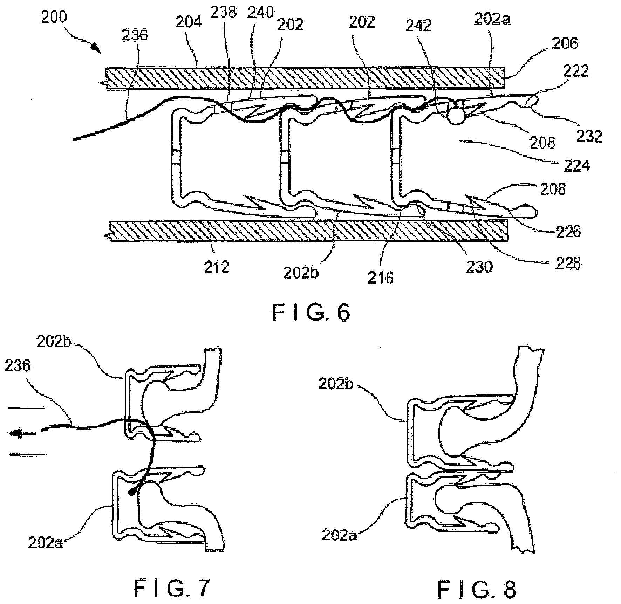

As shown in Figs. 6-8, a device 200 according to another exemplary embodiment may be substantially similar to the device 100 described above. Similarly to the device 100, the device 200 comprises a plurality of clips 202 housed within an insertion member 204 and coupled to one another in a chain-like fashion via distal and proximal locking features 230, 232. The device 200 further comprises a deployment member slidable within a lumen 212 of the insertion member 204 to move the plurality of clips 202 distally relative to the insertion member 204 to deploy a distal-most clip 202a. The clips 202, the insertion member 204 and the deployment
member are substantially similar to the clip 102, the insertion member 104 and the deployment member 110, respectively, described above. In particular, the clip 202 extends from a proximal end 216 to a distal end 222 and includes a channel 224 extending therethrough. Tissue drawn into the channel 224 is held therewithin via arm elements 208 extending from a first end 226 attached to the distal end 222 to a second end 228 extending into the channel 224 and toward the proximal end 216. The device 200, however, is configured to be utilized in tissue approximation procedures such that each of the clips 202 in the clip chain remains connected to one another after deployment via a string 236 or cable.

Alternatively, the clips 202 may be connected to one another via a suture, cable, etc. after they have been clipped to the target portions of tissue. For example, each of the clips 202 may be coupled to one another via a string or suture 236 passing through holes 238 extending laterally through sides 240 of each of the clips 202. A distal end 242 of the string 236 may be coupled to a distal-most clip 202a via, for example, an enlarged end which prevents the distal end 242 of the string 236 from passing therethrough. A proximal end of the string 236 extends through the lumen 212 of the insertion member 204 proximally of a proximal end thereof such that a user of the device 200 may have access to the proximal end of the string 236.

According to another method for tissue approximation using the device 200, the method not forming part of the claimed invention, the device 200 is inserted into a body until a distal end 206 of the insertion member 204 is proximate a first portion of target tissue. Similarly to the device 100, a vacuum or suction force may be applied through the lumen 212 of the insertion member 204 to draw the first portion of target tissue into the channel 224 of the distal-most clip 202a, thereby capturing the first portion of target tissue between the arm elements 208. Once the first portion of target tissue has been captured by the distal-most clip 202a, the distal-most clip 202a is deployed by moving the deployment member distally relative to the insertion member 204 as described above in regard to the device 100.

After the distal-most clip 202a has been deployed, an immediately proximal adjacent clip 202b becomes the distal-most clip and the insertion member is moved to a position in which the clip 202b is positioned over a second portion of target tissue separated from the first portion of target tissue by a distance, as shown in Fig. 7. The second portion of target tissue is drawn into the channel 224 of the clip 202b as described above and captured therein via arm elements 208 and the clip 202b is released from the insertion member. After the clips 202a, 202b have been deployed, the user may pull the proximal end of the string 236 proximally to draw the clips 202a, 202b toward one another. As described above, the distal end 242 is coupled to the distal-most clip 202a in a manner which prevents the string 236 from being disengaged therefrom. Thus, pulling the string 236 proximally draws the clips 202a, 202b toward one another, thereby approximating the first and second portions of target tissue, as shown in Fig. 8.

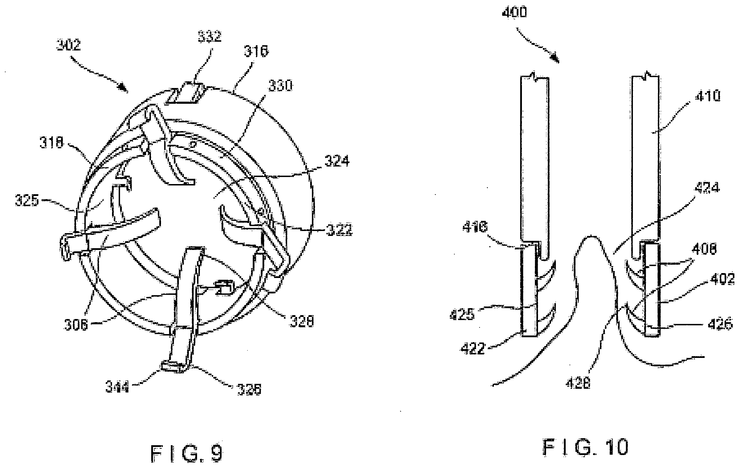

As shown in Fig. 9, a clip 302 according to another exemplary embodiment of the present disclosure is substantially similar to the clip 102 described above in regard to the device 100. Similarly to the clip 102, the clip 302 extends from a proximal end 316 to a distal end 322 and includes a channel 324 extending therethrough. Target tissue is captured in the channel 324 via arm elements 308 extending from a first end 326 to a second end 328. The arm elements 308, are pivotably coupled to the distal end 322 with the second ends 328 of the arm elements 308 received within the channel 324 pointed toward the proximal end 316. Thus, when tissue is received within the channel 324, the arm elements 308 pivot moving the second ends moved radially outward toward a wall 325 of the channel 324 while the first end 326 of each arm element 308 moves radially inward toward a centerline of the channel 324. The first end 326 of any or all of the arm elements 308 may include one or more tissue grasping features 344 (e.g., spikes, pincers, barbs) extending laterally therefrom such that when the first end 326 is moved radially inward toward the centerline of the channel 324, the tissue grasping feature 344 grasps a base portion of the tissue received within the channel 324, capturing the tissue therein.

Similarly to the device 100, the clip 302 also includes distal and proximal locking features 330, 332 so that a plurality of clips 302 may be coupled to one another in a chain-like fashion within an insertion member. The clip 302 may be utilized with an insertion member and deployment member in a manner substantially similar to the insertion member 104 and deployment member 110 described above. Similarly to the device 200, the clip 302 may also include a string or other member connecting a plurality of clips 302 so that the clips 302 may be utilized for tissue approximation procedures, as described above in regard to the device 200.

As shown in Fig. 10, a device 400 not falling within the scope of the claimed invention is substantially similar to the device 100. The device 400 comprises a clip 402 substantially similar to the clip 102, extending from a proximal end 416 to a distal end 422 and including a channel 424 extending therethrough. The clip 402, however, includes arm elements 408 distributed about both a perimeter and a length of an interior surface 425 of the channel 424. For example, a first row of arm elements 408 may be equally distributed about an interior circumference of the clip 402 proximate the distal end 422. A second row or arm elements 408 may also be equally distributed about an interior circumference of the clip 402, positioned proximally of the first row of arm elements 408 and spaced therefrom via a distance. Each of the arm elements 408 extends from a first end 426 attached to the interior surface 425 to a second end 428 extending into the channel 424 and pointed toward the proximal end 416. Similarly to the clip 102, the arm elements 408 may include, for example, barbs, flaps, ridges, spikes. In some variants, the clip 402 may include any number of rows of arm elements 408 extending about a circumference and along a length of the clip 402. In some variants the arm elements 408 are not required to be equally distributed about an interior circumference of the clip 402.

The clip 402 may be utilized with an insertion member (not shown) and deployment member 410 in a manner substantially similar to either of the devices 100, 200 described above. Although the device 400 is shown with a single clip 402 coupled to the deployment member 410, is some alternatives, the device 400 comprises a plurality of clips 402 connected to one another in a chain-like fashion, as described above in regards to the devices 100, 200.

While embodiments have been described above, a number of modifications and changes may be made without departing from the scope of the disclosure. Thus, it is intended that the present disclosure cover modifications and variations provided that they come within the scope of the appended claims and their equivalents.

## Claims

1. A tissue clipping device (100; 200; 300) comprising:
a first body (102a, 102b, 102c; 202a; 302) extending from a proximal end (116; 216; 316) to a distal end (122; 222; 322) and including a first channel (124; 224; 324) extending therethrough;
a plurality of first arm elements (108; 208; 308), each of the plurality of first arm elements (108; 208; 308) extending radially into the first channel (124; 224; 324) from a first end (126; 226; 326) coupled to the first body (102a, 102b, 102c; 202a; 302) to a second end (128; 228; 328) proximal of its first end (126; 226; 326) so that tissue drawn into a distal end of the first channel (124; 224; 324) proximally beyond the second end (128; 228; 328) of the plurality of first arm elements (108; 208; 308) is compressed between each of the plurality of first arm elements (108; 208; 308) and retained in the first channel (124; 224; 324; 324);
a second body (102b, 102c, 102d; 202b; 302) extending from a proximal end (116; 216; 316) to a distal end (122; 222; 322) and including a second channel (124; 224; 324) extending therethrough; a plurality of second arm elements (108; 208; 308), each of the plurality of second arm elements (108; 208; 308) extending radially into the second channel (124; 224; 324) from a first end (126; 226; 326) coupled to the second body (102b, 102c, 102d; 202b; 302) to a second end (128; 228; 328) proximal of its first end (126; 226; 326) so that tissue drawn into a distal end of the second channel (124; 224; 324) proximally beyond the second ends (128; 228; 328) of the plurality of second arm elements (108; 208; 308) is compressed between each of the plurality of second arm elements (108; 208; 308) and retained in the second channel (124; 224; 324); and an insertion element (104; 204) within which the first body (102a, 102b, 102c; 202a; 302) and second body (102b, 102c, 102d; 202b; 302) are slidably received, the first body (102a, 102b, 102c; 202a; 302) including a proximal locking feature (132; 232; 332) and the second body (102b, 102c, 102d; 202b; 302) including a distal locking feature (130; 230; 330) interacting with the proximal locking feature (132; 232; 332) of the first body (102a, 102b, 102c; 202a; 302) to releasably couple the first body (102a, 102b, 102c; 202a; 302) to the second body (102b, 102c, 102d; 202b; 302).

2. The tissue clipping device (100; 200; 300) of claim 1, wherein the plurality of first arm elements (108; 208; 308) are one of curved and angled along lengths thereof to form ramped surfaces that facilitate the passage of tissue proximally therealong.

3. The tissue clipping device (100; 200; 300) of claim 1, wherein the distal locking feature (130; 230; 330) and the proximal locking feature (132; 232; 332) include a groove and a latch.

4. The tissue clipping device (100; 200; 300) of claim 1, wherein the proximal end (116; 216; 316) of the first body (102a, 102b, 102c; 202a; 302) is biased radially outward so that, when the proximal end (116; 216; 316) of the first body (102a, 102b, 102c; 202a; 302) is moved distally out of the insertion element (104; 204), the proximal locking feature (132; 232; 332) of the first body (102a, 102b, 102c; 202a; 302) disengages the distal locking feature (130; 230; 330) of the second body (102b, 102c, 102d; 202b; 302).

5. The tissue clipping device (300) of claim 1, wherein the first arm elements (308) are pivotably coupled to the first body (302) such that tissue drawn proximally into the first channel (324) moves the second ends (328) of the first arm elements (308) radially outward toward an interior surface (325) of the first body (302) while the first ends (326) of the first arm elements (308) move toward a centerline of the first channel (324) to grip tissue received therein.

6. The tissue clipping device (300) of claim 5, wherein the first end (326) of each of the plurality of first arm elements (308) includes a tissue gripping feature (344) including one of a spike, a pincer and a barb.

7. A system for clipping tissue, comprising the tissue clipping device (100; 200; 300) of claim 1, wherein the insertion element (104; 204) includes a lumen (112; 212) extending therethrough; a first clip includes the first body (102a, 102b, 102c; 202a; 302) and the plurality of first arm elements (108; 208; 308), the first clip being slidably received within the lumen (112; 212) ; and a second clip includes the second body (102b, 102c, 102d; 202b; 302) and the plurality of second arm elements (108; 208; 308).

8. The system of claim 7, further comprising a deployment member (110) slidably received within the lumen (112; 212) and extending from a proximal end to a distal end releasably coupled to the proximal locking feature (132; 232; 332) of the second clip.

9. The system of claim 7, wherein the proximal locking feature (132; 232; 332) of the first clip is biased radially outward so that, when the first clip is moved distally out of the lumen (112; 212), the proximal locking feature (132; 232; 332) of the first clip moves out of engagement with the distal locking feature (130; 230; 330) of the second clip, releasing the first clip from the second clip.

10. The system of claim 7, wherein the first end of each of the plurality of first arm elements (308) are pivotably coupled to the distal end (322) of the first clip such that, when tissue is received within the first channel (324), the second end (328) of each of the plurality of first arm elements move toward an interior surface (325) of the first channel (324) and the first end (326) of each of the plurality of first arm elements move toward a centerline of the first channel (324) to grip tissue received therein.

11. The system of claim 7, further comprising a suction device configured to be coupled to the proximal end of the insertion element (104; 204) for applying a vacuum force therethrough to draw tissue into the distal end of the first channel (124; 224; 324).

## Patentansprüche

1. Gewebeclipsetzvorrichtung (100; 200; 300), die aufweist:
einen ersten Körper (102a, 102b, 102c; 202a; 302), der sich von einem proximalen Ende (116; 216; 316) zu einem distalen Ende (122; 222; 322) erstreckt und einen ersten Kanal (124; 224; 324) aufweist, der sich durch ihn erstreckt;
mehrere erste Armelemente (108; 208; 308), wobei sich jedes der mehreren ersten Armelemente (108; 208; 308) in den ersten Kanal (124; 224; 324) von einem mit dem ersten Körper (102a, 102b, 102c; 202a; 302) gekoppelten ersten Ende (126; 226; 326) zu einem zweiten Ende (128; 228; 328) proximal von seinem ersten Ende (126; 226; 326) radial erstreckt, so dass Gewebe, das in ein distales Ende des ersten Kanals (124; 224; 324) über das zweite Ende (128; 228; 328) der mehreren ersten Armelemente (108; 208; 308) hinaus proximal gezogen wird, zwischen jedem der mehreren ersten Armelemente (108; 208; 308) komprimiert und im ersten Kanal (124; 224; 324; 324) festgehalten wird;
einen zweiten Körper (102b, 102c, 102d; 202b; 302), der sich von einem proximalen Ende (116; 216; 316) zu einem distalen Ende (122; 222; 322) erstreckt und einen zweiten Kanal (124; 224; 324) aufweist, der sich durch ihn erstreckt;
mehrere zweite Armelemente (108; 208; 308), wobei sich jedes der mehreren zweiten Armelemente (108; 208; 308) in den zweiten Kanal (124; 224; 324) von einem mit dem zweiten Körper (102b, 102c, 102d; 202b; 302) gekoppelten ersten Ende (126; 226; 326) zu einem zweiten Ende (128; 228; 328) proximal von seinem ersten Ende (126; 226; 326) radial erstreckt, so dass Gewebe, das in ein distales Ende des zweiten Kanals (124; 224; 324) über die zweiten Enden (128; 228; 328) der mehreren zweiten Armelemente (108; 208; 308) hinaus proximal gezogen wird, zwischen jedem der mehreren zweiten Armelemente (108; 208; 308) komprimiert und im zweiten Kanal (124; 224; 324) festgehalten wird; und
ein Einführelement (104; 204), in dem der erste Körper (102a, 102b, 102c; 202a; 302) und zweite Körper (102b, 102c, 102d; 202b; 302) verschiebbar aufgenommen sind, wobei der erste Körper (102a, 102b, 102c; 202a; 302) ein proximales Verriegelungsmerkmal (132; 232; 332) aufweist und der zweite Körper (102b, 102c, 102d; 202b; 302) ein distales Verriegelungsmerkmal (130; 230; 330) aufweist, das mit dem proximalen Verriegelungsmerkmal (132; 232; 332) des ersten Körpers (102a, 102b, 102c; 202a; 302) zusammenwirkt, um den ersten Körper (102a, 102b, 102c; 202a; 302) mit dem zweiten Körper (102b, 102c, 102d; 202b; 302) lösbar zu koppeln.

2. Gewebeclipsetzvorrichtung (100; 200; 300) nach Anspruch 1, wobei die mehreren ersten Armelemente (108; 208; 308) über ihre Längen gekrümmt oder abgewinkelt sind, um Rampenflächen zu bilden, die den Durchgang von Gewebe proximal an ihnen entlang erleichtern.

3. Gewebeclipsetzvorrichtung (100; 200; 300) nach Anspruch 1, wobei das distale Verriegelungsmerkmal (130; 230; 330) und das proximale Verriegelungsmerkmal (132; 232; 332) eine Nut und einen Riegel aufweisen.

4. Gewebeclipsetzvorrichtung (100; 200; 300) nach Anspruch 1, wobei das proximale Ende (116; 216; 316) des ersten Körpers (102a, 102b, 102c; 202a; 302) radial nach außen so vorgespannt ist, dass bei distaler Bewegung des proximalen Endes (116; 216; 316) des ersten Körpers (102a, 102b, 102c; 202a; 302) aus dem Einführelement (104; 204) das proximale Verriegelungsmerkmal (132; 232; 332) des ersten Körpers (102a, 102b, 102c; 202a; 302) außer Eingriff mit dem distalen Verriegelungsmerkmal (130; 230; 330) des zweiten Körpers (102b, 102c, 102d; 202b; 302) kommt.

5. Gewebeclipsetzvorrichtung (300) nach Anspruch 1, wobei die ersten Armelemente (308) mit dem ersten Körper (302) schwenkbar gekoppelt sind, so dass in den ersten Kanal (324) proximal gezogenes Gewebe die zweiten Enden (328) der ersten Armelemente (308) radial nach außen zu einer Innenfläche (325) des ersten Körpers (302) bewegt, während sich die ersten Enden (326) der ersten Armelemente (308) zu einer Mittellinie des ersten Kanals (324) bewegen, um darin aufgenommenes Gewebe zu ergreifen.

6. Gewebeclipsetzvorrichtung (300) nach Anspruch 5, wobei das erste Ende (326) jedes der mehreren ersten Armelemente (308) ein Gewebe ergreifendes Merkmal (344) mit einem Stachel, einer Zange oder einem Widerhaken aufweist.

7. System zum Gewebe-Clippen, das die Gewebeclipsetzvorrichtung (100; 200; 300) nach Anspruch 1 aufweist, wobei das Einführelement (104; 204) ein sich dadurch erstreckendes Lumen (112; 212) aufweist; ein erster Clip den ersten Körper (102a, 102b, 102c; 202a; 302) und die mehreren ersten Armelemente (108; 208; 308) aufweist, wobei der erste Clip im Lumen (112; 212) verschiebbar aufgenommen ist; und ein zweiter Clip den zweiten Körper (102b, 102c, 102d; 202b; 302) und die mehreren zweiten Armelemente (108; 208; 308) aufweist.

8. System nach Anspruch 7, das ferner ein Setzbauteil (110) aufweist, das im Lumen (112; 212) verschiebbar aufgenommen ist und sich von einem proximalen Ende zu einem distalen Ende erstreckt, das mit dem proximalen Verriegelungsmerkmal (132; 232; 332) des zweiten Clips lösbar gekoppelt ist.

9. System nach Anspruch 7, wobei das proximale Verriegelungsmerkmal (132; 232; 332) des ersten Clips so radial nach außen vorgespannt ist, dass sich bei distaler Bewegung des ersten Clips aus dem Lumen (112; 212) das proximale Verriegelungsmerkmal (132; 232; 332) des ersten Clips außer Eingriff mit dem distalen Verriegelungsmerkmal (130; 230; 330) des zweiten Clips bewegt, was den ersten Clip vom zweiten Clip löst.

10. System nach Anspruch 7, wobei das erste Ende jedes der mehreren ersten Armelemente (308) mit dem distalen Ende (322) des ersten Clips schwenkbar gekoppelt ist, so dass sich bei Aufnahme von Gewebe im ersten Kanal (324) das zweite Ende (328) jedes der mehreren ersten Armelemente zu einer Innenfläche (325) des ersten Kanals (324) bewegt und sich das erste Ende (326) jedes der mehreren ersten Armelemente zu einer Mittellinie des ersten Kanals (324) bewegt, um darin aufgenommenes Gewebe zu ergreifen.

11. System nach Anspruch 7, das ferner eine Saugvorrichtung aufweist, die so konfiguriert ist, dass sie mit dem proximalen Ende des Einführelements (104; 204) zum Ausüben einer Vakuumkraft durch dieses hindurch gekoppelt ist, um Gewebe in das distale Ende des ersten Kanals (124; 224; 324) zu ziehen.

## Revendications

1. Dispositif d'attache de tissu (100; 200; 300) comprenant :
un premier corps (102a, 102b, 102c; 202a; 302) s'étendant à partir d'une extrémité proximale (116; 216; 316) jusqu'à une extrémité distale (122; 222; 322) et comprenant un premier canal (124; 224; 324) s'étendant à travers ce dernier;
une pluralité de premiers éléments de bras (108; 208; 308), chacun de la pluralité de premiers éléments de bras (108; 208; 308) s'étendant radialement dans le premier canal (124; 224; 324) à partir d'une première extrémité (126; 226; 326) couplée au premier corps (102a, 102b, 102c; 202a; 302) jusqu'à une seconde extrémité (128; 228; 328) à proximité de sa première extrémité (126; 226; 326) de sorte que le tissu aspiré dans une extrémité distale du premier canal (124; 224; 324) de manière proximale au-delà de la seconde extrémité (128; 228; 328) de la pluralité de premiers éléments de bras (108; 208; 308) est comprimé entre chacun de la pluralité de premiers éléments de bras (108; 208; 308) et retenu dans le premier canal (124; 224; 324);
un second corps (102b, 102c, 102d; 202b; 302) s'étendant à partir d'une extrémité proximale (116; 216; 316) jusqu'à une extrémité distale (122; 222; 322) et comprenant un second canal (124; 224; 324) s'étendant à travers ce dernier; une pluralité de seconds éléments de bras (108; 208; 308), chacun de la pluralité de seconds éléments de bras (108; 208; 308) s'étendant de manière radiale dans le second canal (124; 224; 324) à partir d'une première extrémité (126; 226; 326) couplée au second corps (102b, 102c, 102d; 202b; 302) jusqu'à une seconde extrémité (128; 228; 328) à proximité de sa première extrémité (126; 226; 326) de sorte que le tissu aspiré dans une extrémité distale du second canal (124; 224; 324) de manière proximale au-delà des secondes extrémités (128; 228; 328) de la pluralité de seconds éléments de bras (108; 208; 308) est comprimé entre chacun de la pluralité de seconds éléments de bras (108; 208; 308) et retenu dans le second canal (124; 224; 324); et un élément d'insertion (104; 204) à l'intérieur duquel le premier corps (102a, 102b, 102c; 202a; 302) et le second corps (102b, 102c, 102d; 202b; 302) sont reçus de manière coulissante, le premier corps (102a, 102b, 102c; 202a; 302) comprenant une caractéristique de verrouillage proximale (132; 232; 332) et le second corps (102b, 102c, 102d; 202b; 302) comprenant une caractéristique de verrouillage distale (130; 230; 330) interagissant avec la caractéristique de verrouillage proximale (132; 232; 332) du premier corps (102a, 102b, 102c; 202a; 302) pour coupler, de manière amovible, le premier corps (102a, 102b, 102c; 202a; 302) au second corps (102b, 102c, 102d; 202b; 302).

2. Dispositif d'attache de tissu (100; 200; 300) selon la revendication 1, dans lequel la pluralité de premiers éléments de bras (108; 208; 308) sont incurvés ou coudés le long de leurs longueurs pour former des surfaces à rampe qui facilitent le passage du tissu de manière proximale le long de ces derniers.

3. Dispositif d'attache de tissu (100; 200; 300) selon la revendication 1, dans lequel la caractéristique de verrouillage distale (130; 230; 330) et la caractéristique de verrouillage proximale (132; 232; 332) comprennent une rainure et un verrou.

4. Dispositif d'attache de tissu (100; 200; 300) selon la revendication 1, dans lequel l'extrémité proximale (116; 216; 316) du premier corps (102a, 102b, 102c; 202a; 302) est sollicitée radialement vers l'extrémité de sorte que, lorsque l'extrémité proximale (116; 216; 316) du premier corps (102a, 102b, 102c; 202a; 302) est déplacée de manière distale à l'extérieur de l'élément d'insertion (104; 204), la caractéristique de verrouillage proximale (132; 232; 332) du premier corps (102a, 102b, 102c; 202a; 302) dégage la caractéristique de verrouillage distale (130; 230; 330) du second corps (102b, 102c, 102d; 202b; 302).

5. Dispositif d'attache de tissu (300) selon la revendication 1, dans lequel les premiers éléments de bras (308) sont couplés, de manière pivotante, au premier corps (302) de sorte que le tissu aspiré, de manière proximale, dans le premier canal (324), déplace les secondes extrémités (328) des premiers éléments de bras (308) radialement vers l'extérieur, vers une surface intérieure (325) du premier corps (302) alors que les premières extrémités (326) des premiers éléments de bras (308) se déplacent vers une ligne centrale du premier canal (324) pour saisir le tissu reçu à l'intérieur de ce dernier.

6. Dispositif d'attache de tissu (300) selon la revendication 5, dans lequel la première extrémité (326) de chacun de la pluralité de premiers éléments de bras (308) comprend une caractéristique de préhension de tissu (344) comprenant l'un parmi une pointe, une pince et un ardillon.

7. Système pour attacher un tissu, comprenant le dispositif d'attache de tissu (100; 200; 300) selon la revendication 1, dans lequel l'élément d'insertion (104; 204) comprend une lumière (112; 212) s'étendant à travers ce dernier; une première attache comprend le premier corps (102a, 102b, 102c; 202a; 302) et la pluralité de premiers éléments de bras (108; 208; 308), la première attache étant reçue, de manière coulissante, à l'intérieur de la lumière (112; 212); et une seconde attache comprend le second corps (102b, 102c, 102d; 202b; 302) et la pluralité de seconds éléments de bras (108; 208; 308).

8. Système selon la revendication 7, comprenant en outre un élément de déploiement (110) reçu, de manière coulissante, à l'intérieur de la lumière (112; 212) et s'étendant d'une extrémité proximale à une extrémité distale couplée de manière amovible à la caractéristique de verrouillage proximale (132; 232; 332) de la seconde attache.

9. Système selon la revendication 7, dans lequel la caractéristique de verrouillage proximale (132; 232; 332) de la première attache est sollicitée de manière radiale vers l'extérieur, de sorte que, lorsque la première attache est déplacée de manière distale hors de la lumière (112; 212), la caractéristique de verrouillage proximale (132; 232; 332) de la première attache n'est plus en mise en prise avec la caractéristique de verrouillage distale (130; 230; 330) de la seconde attache, libérant la première attache de la seconde attache.

10. Système selon la revendication 7, dans lequel la première extrémité de chacun de la pluralité de premiers éléments de bras (308) est couplée de manière pivotante à l'extrémité distale (322) de la première attache, de sorte que, lorsque le tissu est reçu à l'intérieur du premier canal (324), la seconde extrémité (328) de chacun de la pluralité de premiers éléments de bras se déplace vers une surface intérieure (325) du premier canal (324) et la première extrémité (326) de chacun de la pluralité de premiers éléments de bras se déplace vers une ligne centrale du premier canal (324) pour saisir le tissu reçu à l'intérieur de ce dernier.

11. Système selon la revendication 7, comprenant en outre un dispositif d'aspiration configuré pour être couplé à l'extrémité proximale de l'élément d'insertion (104; 204) pour appliquer une force de vide à travers ce dernier pour aspirer le tissu dans l'extrémité distale du premier canal (124; 224; 324).
